# EUROPEAN PATENT SPECIFICATION

(11) **EP 1 053 725 B1**
(45) Date of publication and mention of the grant of the patent: **20.04.2005**
(21) Application number: 99201575.0
(22) Date of filing: 21.05.1999
(51) Int. Cl.: A61F 5/445, A61F 13/02, A61F 5/443

(54) **Device for use by ostomy patients**
Vorrichtungen zur Verwendung durch Ostomiepatienten
Dispositifs destinés à l'usage des patients ostomiques

(43) Date of publication of application: 22.11.2000
(73) Proprietor: Eurotec Beheer B.V., 4705 AG Roosendaal (NL)
(72) Inventor: Van der Leden, Arie Gijsbert, 2950 Kapellen (BE)

(56) References cited:
- EP-A- 0 638 301
- US-A- 5 820 578

## Description

A ready to use set of adhesive wedges for filling plies and folds of the uneven peristomal skin consisting of a disk from a skin-friendly adhesive, on one or both sides covered by a release paper and die cut like a tart, thus forming wedge-shaped pieces ideally forming a barrier to fill uneven areas on both sides of a stoma. As these wedges are available in various lengths of radius and in different widths, it will be suitable for stomas positioned in difficult areas such as plies, folds and irregularities of the skin thus forming an extra barrier, which helps to avoid leakage and undermining the skin protective adhesive layer by urine or liquid stool.

A number of diseases involving the intestinal and urinary track may result in the construction of an artificial outlet on the abdominal area for stool and/or urine; a stoma. If the large intestine flows into the abdominal wall one refers to a colostomy. If this is the case with the small intestine one talks about an ileostomy. Last but not least, one refers to a urostomy, if the urine is disposed through an artificial opening in the abdominal wall. During the past years a large variety of stoma appliances have been developed.
People with a colostomy use to collect their rather thick stool in closed pouches, which usually are changed several times a day. People with an ileostomy use to collect their rather liquid stool in pouches with an outlet, which can be opened and closed with a tail clip. People with an urostomy collect their urine in a pouch with a drain tap. Ileostomy and urostomy pouches are usually changed once a day.

Stoma appliances are to be divided in two main systems; the 1-piece and the 2-piece system. In the case of a 1-piece system, the body side wafer - which consists of a special adhesive - forms one integral part with the pouch. This type of pouch is designed to be affixed directly to the skin, and has the advantage that the total surface of the adhesive is flat and flexible, which allows it to follow the contours of the skin. However, the major disadvantage of a 1-piece system is the fact, that the adhesive has to be torn from the skin with every pouch change, which may cause skin irritations.

In the case of a 2-piece stoma system, the body side adhesive and the pouch are two separate components. The stoma pouch is provided with a circular shaped coupling member, which can be attached on or around the projecting rib of the body side flange. A major advantage of a 2-piece stoma system is that the peristomal skin will not be disturbed during pouch changes, as the adhesive part will stay in place. A major disadvantage of a 2-piece stoma system is that the body side adhesive wafer becomes less flexible, because of the rigidity and the higher profile of the in-built coupling member, which makes it less suitable for stomas positioned in difficult areas such as plies, folds and irregularities of the skin.
With both 1-piece and 2-piece stoma systems problems may occur with leakage, especially when retracting the peristomal skin or more or less deep folds near the stoma, therby forming a channel allowing the stool or urine to undermine the adhesive body side wafer, thus causing skin irritation and leakage problems.

In practice one uses different methods to avoid such undermining of a body side wafer by:
1. Using convex inserts or using stoma appliances with a convex adhesive thus creating a better contact with the skin, even in lower peristomal areas.
2. Using an adhesive stoma paste as a filler of irregularities of the skin.
3. Using wedge shaped pieces of skin-friendly preferably double-sided adhesive as a filler of plies and folds.

The method of filling plies and folds in the peristomal skin by means of wedge-shaped pieces of adhesive material is not new and has been widely used since the beginning of 1980. The invention of this method is also claimed by the inventor of this application, but is free to use by anybody.
In practice the problem with thewedge-shaped piece of a stoma adhesive is that each patient needs many of these wedges, each of which has to be cut out by hand from sheets of stoma adhesive materials. As most stoma patients are elderly people with manual and visual handicaps, in practice it is very difficult for them to cut out exactly the same size and shape every time, even when the stoma care nurse has drawn the outlines of the wedges on the release paper.
Moreover, cutting out these wedges is very time consuming and is usually not done very economically, resulting in a lot of expensive waste adhesive material being thrown away.
This patent application does not apply to the shape or the use of an individual wedge, but is applicable to the way the wedges are formed ready-to-use like a tart divided into pieces with inside rounded notches between the intersections of the radius and the circumference.

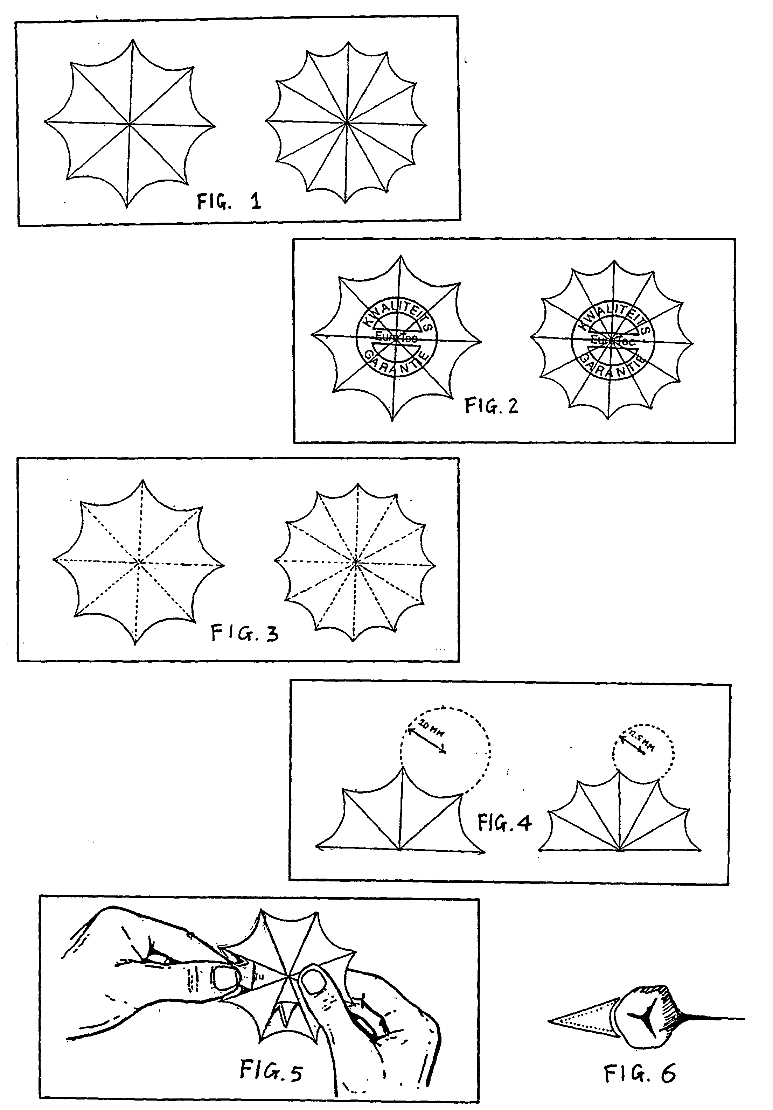

**Fig. 1.** Shows two different sizes and shapes of such a set of pre-cut wedges, one with 8 wedges per disk and one with 12 wedges per disk. However more or fewer wedges per disk is optional.
**Fig. 2.** Shows the other side of the disk, where each radius has been die cut as well, whereby a sticker in the centre keeps the individual wedges together, thereby allowing them to be taken off one by one without falling apart.
**Fig 3.** It is also possible to have one side of the release paper die cut and the opposite release paper kiss cut, so that the individual wedges will stay together without using an extra sticker, as shown in this figure.
**Fig. 4.** Show in detail the inside directed rounded notches between the intersections of each pair of radii with the circumference of the disk. In this case the radii of these notches are in line with the average radius of the most common stoma, in this case 12.5 mm for the model with 12 wedges and 20 mm for the model with 8 wedges, but they might have other diameters too.
**Fig. 5.** Shows how the individual wedges are separated from the disk.
**Fig. 6.** Shows a pair of wedges attached within a peristomal fold of the skin, whereby the smallest wedge is positioned on the bottom of the fold followed by the wider example on top of the smaller one. Should one need to tide over a higher level, one could use several more layers of wedges, using larger or even smaller wedges, which can easily be obtained by cutting an existing wedge into smaller pieces.

## Claims

1. A ready-to-use set of adhesive wedges for filling plies and folds of the uneven peristomal skin, **characterised by** the fact that it consists of a disk shaped stoma adhesive, on one or both sides covered by a release paper and die cut in wedges like a tart, thus forming wedge-shaped pieces of adhesive material, whereby however between the intersections of each radius with the circumference of the disk inside-directed rounded notches have been formed, and as each radius has been totally die cut through the adhesive material on one side of the adhesive disk the individual shaped wedges are kept together by means of a sticker or any other type of bonding, thus allowing each wedge to be taken off one by one without falling apart.

2. A ready-to-use set of adhesive wedges as in claim 1, **characterised by** the fact that the radius of each wedge has been totally die cut through one side of the adhesive disk but has only been kiss cut on the opposite release paper thereby keeping this release paper intact and allowing each wedge to be taken off one by one without falling apart.

3. A ready-to-use set of adhesive wedges as in claim 1, **characterised by** the fact that the smallest side of the formed wedge-shaped pieces of adhesive material - being the connection between the intersections of each radius with the circumference of the adhesive disk - has not been formed by inside-directed rounded notches but by a straight cut or outside-directed rounded bulges.

4. A ready-to-use set of adhesive wedges as in claim 2, **characterised by** the fact that the smallest side of the formed wedge-shaped pieces of adhesive material - being the connection between the intersections of each radius with the circumference of the adhesive disk - has not been formed by inside-directed rounded notches but by a straight cut or outside-directed rounded bulges.

5. A ready-to-use set of adhesive wedges as in claim 1, **characterised by** the fact that the radius of each wedge has been totally die cut through the adhesive disk, whereby the individual shaped wedges are not kept together by means of any other type of bonding, thus allowing each wedge to fall apart and be delivered individually.

## Patentansprüche

1. Fertigsatz von klebenden Keilchen zum Ausfüllen von Falten in einer unebenen peristomalen Haut, **gekennzeichnet durch** die Tatsachse, daß diese aus einer scheibenförmigen Stomaklebeschicht besteht, an einer oder beiden Seiten mit einem Schutzpapier versehen und in Keilchen, wie Tortenschnitten, gestanzt, wodurch keilförmige Teilchen Klebmaterials gebildet werden, wobei jedoch zwischen den Schnittpunkte von jedem Radius mit dem Scheibenumfang nach innen gerichtete runde Aussparungen gebildet sind, wobei, weil jeder Radius ganz **durch** das Klebmaterial an einer Seite der klebenden Scheibe gestanzt ist, die individuell gebildeten Keilchen **durch** einen Selbstkleber oder auf eine andere Verbindungsweise zusammengehalten werden, wodurch jedes Keilchen ein nach dem anderen abgenommen werden kann, ohne ein Auseinanderfallen.

2. Fertigsatz von klebenden Keilchen nach Anspruch 1, **gekennzeichnet durch** die Tatsache daß der Radius jedes Keilchens ganz **durch** eine Seite der klebenden Scheibe gestanzt ist, aber an dem gegenübergelegenen Schutzpapier nur kiss-cut ist, wobei dieses Schutzpapier intakt bleibt, und jedes Keilchen ein nach dem anderen abgenommen werden kann, ohne ein Auseinanderfallen.

3. Fertigsatz von klebenden Keilchen nach Anspruch 1, **gekennzeichnet durch** die Tatsachse daß die kürzeste Seite der keilförmigen Teilchen Klebmaterials - was die Verbindung zwischen den Schnittpunkten jedes Radius mit dem Umfang der klebenden Scheibe ist - nicht **durch** nach innen gerichtete runde Aussparungen, aber **durch** einen rechten Schnitt oder nach außen gerichtete runde Ausstülpungen, gebildet wird.

4. Fertigsatz von klebenden Keilchen nach Anspruch 2, **gekennzeichnet durch** die Tatsachse daß die kürzeste Seite der keilförmigen Teilchen Klebmaterials - was die Verbindung zwischen den Schnittpunkten jedes Radius mit dem Umfang der klebenden Scheibe ist - nicht **durch** nach innen gerichtete runde Aussparungen, aber **durch** einen rechten Schnitt oder nach außen gerichtete runden Ausstülpungen, gebildet wird.

5. Fertigsatz von klebenden Keilchen nach Anspruch 1, **gekennzeichnet durch** die Tatsachse daß der Radius jedes Keilchens ganz **durch** die klebende Scheibe gestanzt ist, wobei die individuell gebildeten Keilchen nicht **durch** einige Verbindungsweise zusammengehalten werden, wodurch ein Auseinanderfallen jedes Keilchens und eine individuelle Ablieferung gestattet werden.

## Revendications

1. Jeu prêt à l'emploi de coins adhésifs destinés à remplir les plis et replis de la peau péristomiale irrégulière, **caractérisé par le fait qu'**il est constitué d'un adhésif pour stoma en forme de disque, recouvert d'un côté ou des deux d'un papier amovible et découpé par poinçonnage comme une tarte, formant ainsi des éléments de matériau adhésif en forme de coin, dans lequel cependant des encoches arrondies dirigées vers l'intérieur ont été formées entre les intersections de chaque rayon avec la circonférence du disque, et comme chaque rayon a été totalement découpé par poinçonnage dans le matériau adhésif sur un côté du disque adhésif, les coins formés individuels sont maintenus ensemble au moyen d'une étiquette ou de tout autre type de liaison, ce qui permet de prélever chaque coin un par un sans dislocation.

2. Jeu prêt à l'emploi de coins adhésifs selon la revendication 1, **caractérisé par le fait que** le rayon de chaque coin a été totalement découpé par poinçonnage dans un côté du disque adhésif, mais a été uniquement kiss-cut sur le papier amovible opposé, pour laisser alors ce papier amovible intact et permettre que chaque coin puisse être prélevé un par un sans dislocation.

3. Jeu prêt à l'emploi de coins adhésifs selon la revendication 1, **caractérisé par le fait que** le côté le plus petit des éléments formés à la forme de coins du matériau adhésif - qui est la liaison entre les intersections de chaque rayon et la circonférence du disque adhésif - n'a pas été formé par des encoches arrondies dirigées vers l'intérieur mais par une découpe droite ou des renflements arrondis dirigés vers l'extérieur.

4. Jeu prêt à l'emploi de coins adhésifs selon la revendication 2, **caractérisé par le fait que** le côté le plus petit des éléments formés à la forme de coins du matériau adhésif - qui est la liaison entre les intersections de chaque rayon et la circonférence du disque adhésif ― n'a pas été formé par des encoches arrondies dirigées vers l'intérieur mais par une découpe droite ou des renflements arrondis dirigés vers l'extérieur.

5. Jeu prêt à l'emploi de coins adhésifs selon la revendication 1, **caractérise par le fait que** le rayon de chaque coin a été entièrement découpé par poinçonnage dans le disque adhésif, les coins formés individuellement n'étant pas maintenus ensemble au moyen de tout autre type de liaison, ce qui permet à chaque coin et d'être détaché et disponible individuellement.
